(19) Europäisches Patentamt
European Patent Office
Office européen des brevets

(11) Publication number: 0 405 463 A2

(12) **EUROPEAN PATENT APPLICATION**

(21) Application number: 90112158.2

(22) Date of filing: 26.06.90

(51) Int. Cl.5: A61K 37/02, A61K 47/42

The applicant has subsequently filed a sequence listing and declared, that it includes no new matter.

(30) Priority: 27.06.89 JP 164571/89

(43) Date of publication of application: 02.01.91 Bulletin 91/01

(84) Designated Contracting States: DE FR GB IT

(71) Applicant: **AJINOMOTO CO., INC.**
5-8, Kyobashi 1-chome, Chuo-ku
Tokyo 104(JP)

Applicant: **Kishimoto, Tadamitsu**
3-5-1, Nakano
Tondabayashi-shi, Osaka-fu(JP)

(72) Inventor: **Kishimoto, Tadamitsu**
No. 3-5-1 Nakano, Tondabayashi-shi
Osaka-fu(JP)
Inventor: **Hirano, Toshio**
No. 19-C-501 Nihogaoka
Ibaraki-shi, Osaka-fu(JP)
Inventor: **Akiyama, Yukio**, c/o Central Research Laboratories
Ajinomoto Co. Inc., No. 1-1,Suzuki-cho
Kawasaki-ku
Kawasaki-shi, Kanagawa-ken(JP)
Inventor: **Okano, Akira**, c/o Central Research Laboratories
Ajinomoto Co. Inc., No. 1-1,Suzuki-cho
Kawasaki-ku
Kawasaki-shi, Kanagawa-ken(JP)

(74) Representative: **Strehl, Schübel-Hopf, Groening**
Maximilianstrasse 54 Postfach 22 14 55
D-8000 München 22(DE)

(54) **Supporting agents for anti-cancer therapy.**

(57) Human B cell, differentiation factor or its biological equivalent is used for preparing a supporting agent for anti-cancer therapy.

EP 0 405 463 A2

## SUPPORTING AGENTS FOR ANTI-CANCER THERAPY

The present invention relates to the use of human BCDF as an effective ingredient for the production of supporting agents for anti-cancer therapy.

Factors which differentiate mature B cells into antibody-producing cells in humans and mice are collectively called B cell differentiation factor (BCDF).

The present inventors made investigations on human BCDF having such an important action in vivo in human and succeeded in determining its DNA sequence and amino acid sequence (Japanese Patent Applications OPI 42688/1988 and 56291/1988). Further, human BCDF has been successfully produced using E . coli (Japanese Patent Application OPI 157996/1988). The present inventors also found that human BCDF can be used as immunotherapeutic agent effective for primary and secondary immunodeficiency syndrome, as a supporting agent effective for bone marrow transplantation therapy, as an agent for potentiating the vaccine effect and can be a therapeutic agent for thrombocytopenia (Japanese Laid-Open Patent Appl. No. 63524/1989, Japanese Patent Application Nos. 183083/1988, 310578/1988 and 6954/1989).

It was also proposed to designate human BCDF as BSF-2 or interleukin 6 (IL-6) (Nature, 324 , 73 (1986) , EMBO. J. 6, 1219 (1987)). In the present specification, however, the conventional term "BCDF" is used. Furthermore, human BCDF as used according to the present invention has no interferon activity and is thus different from IFN-$\beta_2$ having interferon activity (Published European patent application no. 0220574).

However, it has not been heretofore reported that this human BCDF has a pharmacological effect as a supporting agent for anti-cancer therapy.

Firstly, cancers against which the potent supporting agent for anti-cancer therapy containing human BCDF and complications associated therewith are outlined herein and at the same time, currently applied therapy is also briefly explained.

In anti-cancer therapy, it is strongly desired to establish an intensive anti-cancer treatment.

In order to cure, e.g., acute leukemia which is one of cancers, it is an essential requirement to achieve complete remission. For this purpose, it is necessary to administer a potent anti-cancer chemotherapeutic agent in a large dose at the initial stage of the therapy. However, such an intensive therapy causes as an extremely serious side effect, the inhibition of the proliferation of bone marrow cells (hereafter referred to as bone marrow dysfunction). Because of this side effect, so-called chemotherapeutic death may be caused sometimes and hence such intensive therapy has not been positively performed (Journal of the Japan Hematological Society, 52 (2), 207 (1989)).

Furthermore, administration of anti-cancer agents against lung cancer, etc. also causes infections or bleeding due to decrease of the number of leucocytes or platelets and therefore, a 3-week-one-cycle therapy should be applied, in which medication is discontinued for about 3 weeks until the count of peripheral blood cells is recovered to normal levels, whereafter the carcinostatic agent is again administered.

Also in radiotherapy, the radiation irradiated cannot be applied frequently and/or in a large dose, because of similar problems.

The side effect caused by anti-cancer therapy-induced bone marrow dysfunction is attributable to the decrease and reduction in number and function of granulocytes or macrophages having killing activites against infectious bacteria.
Furthermore, even normal cells are killed by the anti-cancer chemotherapeutic agent, such as blood immune cells in charge of vital protection, such as B lymphocytes capable of producing antibodies against infectious bacteria. The reduction of these immunological and hematological functions readily may cause pneumonia or septicemia by E . coli , Pseudomonas aeruginosa , etc. In these cases, the patient's ability of protecting himself against infections in the whole body and in the organ which is the infected site is important as an incident factor. For example, septicemia is mainly attributed to endogeneous infection from the intestinal mucosa and it is said that even though an antibiotic is used and bacteria in the air are removed, the effectiveness would be poor.

Under the above described actual situation, it is considered that by increasing the count of various blood cells or enhancing the functions of blood cells such as antibody productivity, activated oxygen productivity, etc. or by using supporting agents for accelerating recovery, the therapeutic effect of anti-cancer treatment is enhanced and a potent anti-cancer therapy is intensively performed.

It was reported that a variety of colony stimulating factors, for example, GM-CSF, G-CSF, M-CSF, etc., accelerate recovery of leucocyte count (Progress in Growth Factor Research, 1 , 1 (1989)).

However, there has not yet been found any supporting agent for anti-cancer therapy which enhances both the count of hematocytes in blood and in organs and the functions of activated oxygen productivity, antibody productivity, etc. by *in vivo* administration, thus protecting host from infections and further having the effect of increasing the life span when an intensive anti-cancer therapy is actually applied.

The problem to be solved by the present invention is therefore to provide a supporting agent for anti-cancer therapy which enables to perform an intensive anti-cancer therapy by *in vivo* administration which could not be applied before.

As a result of extensive investigations to solve the foregoing problem, the present inventors have found that human BCDF may effectively be used as an active agent for supporting anti-cancer therapy.

Subject matter of the present invention is therefore the use of human B-cell differentiation factor or its biological equivalent for preparing a supporting agent for anti-cancer therapy.

It was found that the administration of the supporting agent for anti-cancer therapy prepared according to the present invention, which comprises human BCDF (abbreviated as h-BCDF) after radiotherapy or after chemotherapy has the following effects:

(1) it accelerates recovery of leucocyte count in peripheral blood or in organ;
(2) enhances antibody productivity and activated oxygen productivity;
(3) increases the ability of protecting bacterial infections; and,
(4) results in an increased life span effect in mice after intensive radiotherapy or after large dose chemotherapy.

These effects of BCDF allow an increase of the frequency or dose of the anti-cancer treatment.

Human BCDF used in accordance with the present invention has the following amino acid sequence (I) or (II) as shown in Japanese Patent Application Laid-Open Nos. 42688/1988, 56291/1988 and 63524/1989.

Amino acid sequence (I):

Pro Val Pro Pro Gly Glu Asp Ser Lys Asp Val
Ala Ala Pro His Arg Gln Pro Leu Thr Ser Ser
Glu Arg Ile Asp Lys Gln Ile Arg Thr Ile Leu
Asp Gly Ile Ser Ala Leu Arg Lys Glu Thr Cys
Asn Lys Ser Asn Met Cys Glu Ser Ser Lys Glu
Ala Leu Ala Glu Asn Asn Leu Asn Leu Pro Lys
Met Ala Glu Lys Asp Gly Cys Phe Gln Ser Gly
Phe Asn Glu Glu Thr Cys Leu Val Lys Ile Ile
Thr Gly Leu Leu Glu Phe Glu Val Tyr Leu Glu
Tyr Leu Gln Asn Arg Phe Glu Ser Ser Glu Glu
Gln Ala Arg Ala Val Gln Met Ser Thr Lys Val
Leu Ile Gln Phe Leu Gln Lys Lys Ala Lys Asn
Leu Asp Ala Ile Thr Thr Pro Asp Pro Thr Thr
Asn Ala Ser Leu Leu Thr Lys Leu Gln Ala Gln
Asn Gln Trp Leu Gln Asp Met Thr Thr His Leu
Ile Leu Arg Ser Phe Lys Glu Phe Leu Gln Ser
Ser Leu Arg Ala Leu Arg Gln Met

or,

Amino acid sequence (II)

Ala Pro Val Pro Pro Gly Glu Asp Ser Lys Asp
Val Ala Ala Pro His Arg Gln Pro Leu Thr Ser
Ser Glu Arg Ile Asp Lys Gln Ile Arg Tyr Ile
Leu Asp Gly Ile Ser Ala Leu Arg Lys Glu Thr
Cys Asn Lys Ser Asn Met Cys Glu Ser Ser Lys
Glu Ala Leu Ala Glu Asn Asn Leu Asn Leu Pro
Lys Met Ala Glu Lys Asp Gly Cys Phe Gln Ser
Gly Phe Asn Glu Glu Thr Cys Leu Val Lys Ile
Ile Thr Gly Leu Leu Glu Phe Glu Val Tyr Leu
Glu Tyr Leu Gln Asn Arg Phe Glu Ser Ser Glu
Glu Gln Ala Arg Ala Val Gln Met Ser Thr Lys
Val Leu Ile Gln Phe Leu Gln Lys Lys Ala Lys
Asn Leu Asp Ala Ile Thr Thr Pro Asp Pro Thr
Thr Asn Ala Ser Leu Leu Thr Lys Leu Gln Ala
Gln Asn Gln Trp Leu Gln Asp Met Thr Thr His
Leu Ile Leu Arg Ser Phe Lys Glu Phe Leu Gln
Ser Ser Leu Arg Ala Leu Arg Gln Met

The amino acid sequence (II) is the polypeptide added with one Ala at the N terminus of human BCDF (I) (hereafter referred to as human Ala-BCDF). However, human BCDF used in the present invention does not necessarily take the structure shown by the amino acid sequence (I) or (II) described above.

That is, derivatives having a structure in which one or more amino acids are added at the N terminus and/or C terminus of human BCDF (I) or having a structure in which one or more amino acids in the natural human BCDF structure (I) are substituted by other amino acids may also be used as human BCDF of the present invention, so long as they have human BCDF activity, and hence may be considered as a biological equivalent of human BCDF. Preferably, human BCDF (I) or human Ala-BCDF (II) is employed. The content of human BCDF in accordance with the present invention is 0.0001 to 100 wt%, preferably 0.1 to 1.0 wt%, based on the overall weight of the supporting agent for anti-cancer therapy.

In addition, the supporting agent for anti-cancer therapy of the present invention which comprises human BCDF as an effective ingredient may also contain a stabilizer such as serum albumin, etc. a carrier such as mannitol, etc. and furthermore, the supporting agent for anti-cancer therapy of the present invention may also contain, in addition to human BCDF, one or more anti-cancer chemotherapeutic agents as aids.

An example of the anti-cancer chemotherapeutic agent is 5-fluorouracil (hereafter refered to as 5-FU). In addition, any other agents including mitomycin C, cyclophosphamide, adriamycin, VP-16, Ara-C, etc. which possibly inhibit proliferation of normal bone marrow cells may also be used.

The amount of these aids added is not particularly limited but when the amount of human BCDF is taken as 100 parts by weight, each of them may be incorporated in an amount of 100 to 200,000 parts by weight.

It is to be noted that the added amount of these aids is not limited to the aforesaid range but may be appropriately varied depending upon the condition, the age of the patient, etc.

The aids such as 5-FU; etc. need not always be administered as similar drugs at the same time when human BCDF is administered. That is, these aids may also be administered at an appropriate stage before or after administration of the supporting agent for anti-cancer therapy comprising human BCDF as an effective ingredient.

Of course, the supporting agent for anti-cancer therapy of the present invention may be administered in combination with other cytokines, for example IL-3, G-CSF, GM-CSF, M-CSF, IL-1, etc. and immunotherapeutic agents, for example, lentinan. The supporting agent for anti-cancer therapy may be administered in any route that is, it may be injected intravenously, intramuscularly or subcutaneously.

It is also desired to administer the supporting agent after anti-cancer therapy but the supporting agent may also be administered prior to anti-cancer therapy or simultaneously.

An appropriate dose of human BCDF is 0.001 to 1000 µg/kg, preferably 0.01 to 500 µg/kg, more preferably 0.1 to 250 µg/kg. However, the dose is not limited to the range described above but may be appropriately determined depending upon the condition of patient.

Human BCDF used in the present invention may be any of the products produced and purified from human T cells, B cells, fibroblasts, etc. by known methods (Proc. Natl. Acad. Sci. USA, 82 , 5490 (1985)) and human BCDF produced by culturing a transformant obtained by introducing a gene encoding human BCDF by transformation into an appropriate host such as E . coli , yeast, monkey cells (COS cells), hamster cells, bacteria, etc. using an appropriate vector and by further purification may also be used.

With respect to details of these processes, reference is made to Laid-Open Japanese Patent Applications 115024/1986, 42688/1988, 56291/1988 and 157996/1988.

Effects

The supporting agents for anti-cancer therapy prepared by the use of human BCDF according to the present invention exhibits activity of restoring immunological and hematological functions in peripheral blood and/or organs after anti-cancer therapy such as radiotherapy, chemotherapy, etc. and of inhibiting proliferation of endogeneous infectious bacteria. That is, the supporting agent inhibits side effects in intensive anti-cancer therapy and causes the effect of an increased life span and thereby makes a potent anti-cancer therapy possible. Therefore, the supporting agent is effective for the treatment of cancer patients.

Hereafter the present invention is explained with reference to the examples.

Example 1

Preparation of human BCDF

The HPLC fraction (50 - 60% acetonitrile solution containing 0.1% TFA) of human BCDF (I) or human Ala-BCDF (II) was replaced in PBS solution by gel filtration. A suitable amount of 20% human serum albumin solution (containing 3.3 mg/ml of sodium, 3.1 mg/ml of chlorine, 2.659 mg/ml of sodium caprylate and 4.2925 mg/ml of sodium acetyltryptophane) was added thereto and the mixture was aseptically filtered to make "human BCDF preparation". For administration to mouse, a preparation using mouse serum albumin or mouse serum instead of human serum albumin was also prepared. In the following examples, such a preparation is also referred to as "human BCDF preparation".

Example 2

Effect of immunological and hematological recovery of radiation-exposed mice by administration of the human BCDF preparation.

A miniosmotic pump (manufactured by ALZET Model 2001) packed with 70 µg of the human BCDF preparation or 70 µg of human serum albumin for control was embedded under the skin of DBA/2 strain 18 mice (female, age of 10 weeks) exposed to 4.0 Gy (0.5 Gy/min) of X rays, respectively, thereby to administer the preparation, while slowly releasing the ingredient over 7 days.

Firstly, blood was collected from the respective groups with passage of time and the count of hematocytes was measured with an automatic hematocytometer (manufactured by TOA Medical Electron Co., Model K-1000). As shown in Table 1, a promoted recovery of leucocyte count was significantly noted 10 to 15 days after the exposure in the group to which the human BCDF preparation had been administered. Furthermore, a blood smear specimen was stained by the May-Grünwald-Giemsa method and its differentiation was measured; among leucocytes, an increase of granulocytes, and immature myeloid cells, such as myelocytes was especially recognized. The count of erythrocytes was not greatly different and the count of platelets was increased by administration of the human BCDF preparation.

Table 1

| Days After Exposure | Administration of Human BCDF Preparation | Count of Leucocytes (/µl) | Count of Lymphocytes (/µl) | Count of Granulocytes (/µl) | Count of immature myeloid cells (/µl) |
|---|---|---|---|---|---|
| 7 | + | 1970±410 | 390±270 | 1480±110* | 10± 10 |
| | - | 1600±370 | 480±310 | 1080± 50 | 30± 40 |
| 10 | + | 2330±210* | 950±160 | 970±170* | 420±240* |
| | - | 1600±300 | 920±320 | 580± 10 | 30± 30 |
| 15 | + | 4730±310* | 2000±230 | 2680± 60* | 100±100 |
| | - | 3030±410 | 1690±220 | 1260±220 | 20± 30 |
| Normal value | | 3000±700 | 2300±480 | 620±230 | 0 |

*: significant difference in p = 5%

In addition, the count of hematopoietic precursor cells in the spleen was also examined 7 days after the exposure.

The count of stem cells (CFU-S) was measured by the spleen colony forming method according to Till et al. (Rad. Res., 14 , 213 (1961)); the count of each of pluripotential stem cells (CFU-GEM), granulocyte macrophage precursor cells (CFU-GM) and erythroblast precursor cells (premature type: BFU-E, mature type: CFU-E) was measured by the methyl cellulose method (Experimental Method for Hematopoietic Stem Cells, CHUGAI MEDICAL Co. (1986)) after adding 2.5% pokeweed mitogen-stimulated mouse spleen cell culture supernatant and 2 units/ml of erythropoietin; and the count of megakaryocyte precursor cells (CFU-Meg) was measured by culturing by the fibrin clot method followed by acetylcholine esterase staining (Exp. Hematol. 15 , 896 (1987)). As shown in Table 2, significant recovery of the count of hematopoietic precursor cells was also noted in the group administered with the human BCDF preparation. This recovery accelerating effect was also noted 10 days after the administration. The effect was similarly recognized also in bone marrow cells.

Table 2

| | Recovery of Precursor Cell Count (when normal value is made 100) | |
|---|---|---|
| Precursor Cells | Group Administered with Human BCDF Preparation | Control Group |
| CFU-S | 50± 0** | 11±0.3 |
| CFU-GEM | 165±10* | 41±26 |
| CFU-GM | 97±11** | 40± 2 |
| BFU-E | 51±13* | 18± 1 |
| CFU-E | 101±21* | 24±0.2 |
| CFU-Meg | 80± 4** | 26± 4 |

*: significant difference in p = 5%
**: significant difference in p = 1%

Using 1 x $10^6$ sheep red blood cells (SRBC) as antigen, sensitization was performed on the day when radiation was irradiated and anti-SRBC antibody productivity was determined in the spleen 7 days after the exposure by the method described in J. Immunol., 141 , 3072 (1988). As shown in Table 3, significant potentiation of the antibody productivity was noted in the group administered with the human BCDF preparation, with respect to both subclasses of IgM and IgG.

In addition, 14 days after the exposure, anti-SRBC antibody titer in serum was also increased by about 4 times, by administration of the human BCDF preparation.

Table 3

| | Count of Anti-SRBC Antibody-Producing Cell (/spleen) (when SRBC-sensitized normal mouse is made 100) | |
|---|---|---|
| Antibody Subclass | Group Administered with BCDF Preparation | Control Group |
| IgM | 145± 28* | 21±1 |
| IgG | 491±104* | 88±1 |

*: significant difference in p = 5%

As stated above, administration of the human BCDF preparation acclerated recovery in the count of leucocyte, the count of hematopoietic precursor cells, the count of antibody-producing cells, etc. in the radiation-exposed mice.

Example 3

Effect of increasing life span by administration of the human BCDF preparation to large-dose radiation-exposed mice

After 10 mice of DBA/2 strain (female, age of 9 weeks, weighing 19 to 22 g) was exposed to 10.5 Gy of X rays (0.5 Gy/min), the human BCDF preparation (10 µg/day) or modified human BCDF preparation (10 µg/day) inactivated by heat treatment of human BCDF at 100°C for 40 minutes for control was subcutaneously administered while slowly releasing the ingredient over 7 days by using miniosmotic pumps, whereby the effect of increasing life span was examined.

As shown in Table 4, administration of the human BCDF preparation showed a significant effect of increasing life span, as compared to the control group.

Table 4

| | Survival Rate for 21 Days (%) |
|---|---|
| Group administered with human BCDF preparation | 60 |
| Control | 0 |

Example 4

Effect of immunological and hematological recovery in 5-FU-administered mice by administration of the human BCDF preparation

A miniosmotic pump (manufactured by ALZET, Model 2001) packed with 70 µg (35,000 units) of the human BCDF preparation or 70 µg of human serum albumin for control was embedded under the skin of DBA/2 strain 24 mice (female, age 10 weeks) about 5 hours after intravenous injection of 5-FU (manufactured by KYOWA HAKKO K.K., 150 mg/ml), respectively, thereby to slowly release the ingredient (10 µg/mouse/day) over 7 days. Each organ was examined with passage of time. As shown in Table 5, an increase of spleen cells (increase in spleen weight) was noted 9 days after administration of 5-FU, by administration of the human BCDF preparation. By cyto-centrifugation of spleen cells, smear specimen was prepared and stained by the May-Grünwald-Giemsa method. As the result, an increase in the number of granulocytes and monocytes was recognized.

Table 5

| Administration of Human BCDF Preparation | Weight of Spleen (mg) |
|---|---|
| + | 162±23* |
| - | 116±17 |

*: significant difference in p = 5%

Furthermore, the counts of hematopoietic stem cell (CFU-S) and granulocyte/macrophage hematopoietic precursor cells (CFU-GM) were determined by the method described in Japanese Patent Application No. 310578/1988 and Biochem. Biophys. Res. Comm., 159 , 933 (1989). Even at the stage as early as 5 to 9 days after administration of 5-FU, promoted recovery was noted in the group administered with the human BCDF preparation (Table 6).

In the peripheral blood, an increase of the number of platelets was noted after 9 to 12 days.

Table 6

| Days After Administration of 5-FU | Administration of Human BCDF Preparation | Count of CFU-S (/spleen) | Count of CFU-GM (/spleen) |
|---|---|---|---|
| 5 | + | 6,400± 3,700* | 1,100± 500 |
| | - | 1,700± 900 | 1,100± 700 |
| 7 | + | 13,400± 2,700* | 30,200± 300* |
| | - | 8,400± 1,000 | 7,100± 500 |
| 9 | + | 307,500±45,600* | 227,800±57,000* |
| | - | 163,600±16,400 | 106,300± 8,200 |

*: significant difference in p = 5%

In addition, sheep red blood cells (SRBC) were administered in 1 x $10^6$ to 1 x $10^8$ on the day when 5-FU was administered and anti-SRBC antibody (IgM) productivity in the spleen was determined with passage of time, according to the method described in J. Immunol., 141 , 3072 (1988). On Day 5 or after, significantly high antibody production was noted in the group administered with the human BCDF preparation (Table 7).

From the foregoing results, it has been established that administration of the human BCDF preparation accelerates the recovery of immunological and hematological functions in mice, which functions were reduced by administration of anti-cancer chemotherapeutic agents.

Table 7

| Days After Administration of 5-FU | Administration of Human BCDF Preparation | Count of Anti-SRBC Antibody Producing Cells (/spleen x $10^{-4}$) |
|---|---|---|
| 5 | + | 1.95* |
| | - | 0.78 |
| 7 | + | 1.59* |
| | - | 0.61 |
| 10 | + | 1.75* |
| | - | 0.20 |

*: significant difference in p = 5%

Example 5

Increase of the life span of mice administered with 5-FU in large dose by administration of the human BCDF preparation

As a model of intensive anti-cancer therapy, 5-FU was intravenously administered to 51 mice of DBA/2 strain (female, age of 10 weeks) in a dose of 300 mg/kg which exceeded $LD_{50}$. In a manner similar to the method of Example 2, the human BCDF preparation was subcutaneously administered to 28 mice and human serum albumin to 23 mice, while slowly releasing the ingredient over 7 days by using miniosmotic pumps. As shown in Fig. 1, 75% of the mice were survived for 21 days or longer in the group administered with the human BCDF preparation, as compared to the control group (survival rate for 21 days: 26%). This life span increasing effect was statistically significant in p of 0.1%, by the $X^2$ assay.

The test revealed that the human BCDF preparation showed a life span increasing effect on mice after the intensive anti-cancer chemotherapy.

Example 6

Protection against infection by administration of the human BCDF preparation

The human BCDF preparation or human serum albumin for control was administered to mice intravenously injected with 300 mg/kg of 5-FU, in a manner similar to the method shown in

Example 5.

On Days 7 to 9 immediately before administration when a significant difference in survival rate appeared between the group administered with the human BCDF preparation and the control group, the liver and spleen in the two groups were homogenated and the count of infected bacteria in the organ was determined by the agar colony method. In any of the groups, endogeneous infection with E . coli was caused but in the group administered with the human BCDF preparation, the count of E . coli was inhibited to 10% or less of that in the control group.

At this point of time,the quantity of the antibody (IgG) to E . coli in mouse serum 9 days after administration was determined in a conventional manner by the sandwich ELISA method, using E . coli - homogenated antigen as solid phase. The group administered with the human BCDF preparation showed anti-E . coli antibody titer as high as about 32 times (group administered with the human BCDF preparation: 2048 units, control group: 32 units; standard anti-E . coli serum was made one unit). In addition, the isolated E . coli was cultured in 100 µl of medium added with mouse serum in a final concentration of 10% and the count of vital cells was then counted by the colony method. Compared to the case where mouse serum from normal mouse or from the control group was added, proliferation of bacteria was inhibited to about 20% by the addition of mouse serum in the group administered with the human BCDF preparation (addition of serum from the group administered with the human BCDF preparation: 5.3, addition of serum from the control group: 27.2 (times, x $10^{-4}$)).

Furthermore, by administration of the human BCDF preparation, activated oxygen production in the organ was also promoted. That is, normal mouse spleen cells were stimulated with phorbol myristate acetate in a conventional manner and the intensity of emission was determined by the chemical emission method using luminol. In the mice administered with the human BCDF preparation, the count of spleen cells was increased and at the same time, emission intensity per unit cell was also increased to more than two-fold (human BCDF preparation: 165 ± 21, control group: 75 ± 31/$10^7$ spleen cells). This effect was also noted not only in normal mice but also in mice after radiotherapy and anti-cancer chemotherapy.

As stated above, administration of the human BCDF preparation induced the acceleration of specific antibody production and activated oxygen production and inhibited growth of endogeneously infected bacteria after intensive anti-cancer therapy.

Brief Description of Drawing

Fig. 1 is a drawing showing that administration of the human BCDF preparation exhibits the effect of increasing life span in mice administered with 5-FU in a large dose.

## Claims

1. The use of human B cell differentiation factor or its biological equivalent for preparing a supporting agent for anti-cancer therapy.
2. The use according to claim 1, wherein said human BCDF has the following amino acid sequence (I).

Amino acid sequence (I):

Pro Val Pro Pro Gly Glu Asp Ser Lys Asp Val
Ala Ala Pro His Arg Gln Pro Leu Thr Ser Ser
Glu Arg Ile Asp Lys Gln Ile Arg Thr Ile Leu
Asp Gly Ile Ser Ala Leu Arg Lys Glu Thr Cys
Asn Lys Ser Asn Met Cys Glu Ser Ser Lys Glu
Ala Leu Ala Glu Asn Asn Leu Asn Leu Pro Lys
Met Ala Glu Lys Asp Gly Cys Phe Gln Ser Gly
Phe Asn Glu Glu Thr Cys Leu Val Lys Ile Ile
Thr Gly Leu Leu Glu Phe Glu Val Tyr Leu Glu
Tyr Leu Gln Asn Arg Phe Glu Ser Ser Glu Glu
Gln Ala Arg Ala Val Gln Met Ser Thr Lys Val
Leu Ile Gln Phe Leu Gln Lys Lys Ala Lys Asn
Leu Asp Ala Ile Thr Thr Pro Asp Pro Thr Thr
Asn Ala Ser Leu Leu Thr Lys Leu Gln Ala Gln
Asn Gln Trp Leu Gln Asp Met Thr Thr His Leu
Ile Leu Arg Ser Phe Lys Glu Phe Leu Gln Ser
Ser Leu Arg Ala Leu Arg Gln Met

3. The use according to claim 1, wherein said human BCDF has the following amino acid sequence (II).

Amino acid sequence (II):

Ala Pro Val Pro Pro Gly Glu Asp Ser Lys Asp
Val Ala Ala Pro His Arg Gln Pro Leu Thr Ser
Ser Glu Arg Ile Asp Lys Gln Ile Arg Tyr Ile
Leu Asp Gly Ile Ser Ala Leu Arg Lys Glu Thr
Cys Asn Lys Ser Asn Met Cys Glu Ser Ser Lys
Glu Ala Leu Ala Glu Asn Asn Leu Asn Leu Pro
Lys Met Ala Glu Lys Asp Gly Cys Phe Gln Ser
Gly Phe Asn Glu Glu Thr Cys Leu Val Lys Ile
Ile Thr Gly Leu Leu Glu Phe Glu Val Tyr Leu
Glu Tyr Leu Gln Asn Arg Phe Glu Ser Ser Glu
Glu Gln Ala Arg Ala Val Gln Met Ser Thr Lys
Val Leu Ile Gln Phe Leu Gln Lys Lys Ala Lys
Asn Leu Asp Ala Ile Thr Thr Pro Asp Pro Thr
Thr Asn Ala Ser Leu Leu Thr Lys Leu Gln Ala
Gln Asn Gln Trp Leu Gln Asp Met Thr Thr His
Leu Ile Leu Arg Ser Phe Lys Glu Phe Leu Gln
Ser Ser Leu Arg Ala Leu Arg Gln Met

4. The use according to any of the claims 1 to 3, wherein said human BCDF has no sugar chain.

5. The use according to any of the claims 1 to 4, wherein said human BCDF is produced in procaryote.

6. The use according to any of the claims 1 to 5, wherein said supporting agent for anti-cancer therapy contains, in addition to human BCDF, at least one anti-cancer chemotherapeutic agent as an aid.

Figure 1

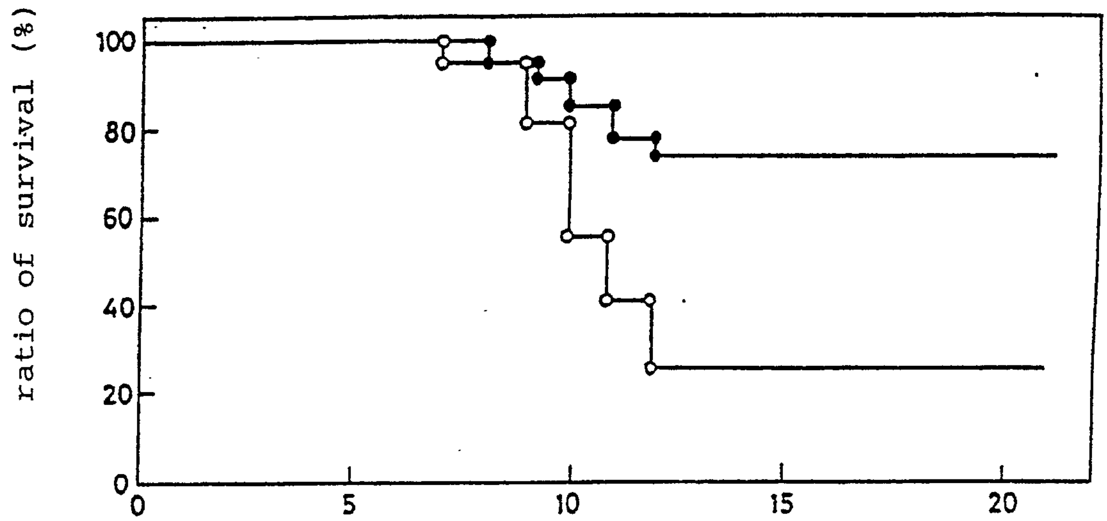


days after administration of 5-FU

○ control

● administration of human BCDF preparation